# EUROPEAN PATENT SPECIFICATION

(11) **EP 1 894 973 B1**
(45) Date of publication and mention of the grant of the patent: **10.06.2009**
(21) Application number: 05739143.5
(22) Date of filing: 28.04.2005
(51) Int. Cl.: C08L 39/06, C01B 31/02, C08K 3/04

(54) **PROCESS FOR PRODUCING PVP-FULLERENE COMPLEX AND AQUEOUS SOLUTION THEREOF**
VERFAHREN ZUR HERSTELLUNG EINES PVP-FULLEREN-KOMPLEXES UND WÄSSRIGE LÖSUNG DAVON
PROCEDE DE FABRICATION D'UN COMPLEXE PVP-FULLERENE ET SOLUTION AQUEUSE DE CE COMPLEXE

(43) Date of publication of application: 05.03.2008
(73) Proprietor: Vitamin C60 Bioresearch Corporation, Tokyo 100-0005 (JP)
(72) Inventor: TAKADA, H., Hiroshima Prefectural University, Shobara-shi, Hiroshima 727-0023 (JP); MATSUBAYASHI, K., Mitsubishi Corporation, Tokyo 100-0005 (JP)
(74) Representative: Calamita, Roberto
(86) International application number: PCT/JP2005/008560
(87) International publication number: WO 2006/117877

(56) References cited:
- JP-A- 8 262 512
- JP-A- 2004 182 771
- JP-A- 2004 267 972
- JP-A- 2005 035 809
- JP-A- 2005 060 380
- Y. N. YAMAKOSHI ET AL.,: "Solubilization of Fullerenes into Water with Polyvinylpyrrolidone Applicable to Biological Tests" J. CHEM. SOC., CHEM. COMMUN., 1994, pages 517-518, XP009099482

## Description

### Technical Field

The invention of this application relates to a novel method of producing an aqueous solution of a fullerene which draws attention as a compound having a bioactivity such as an antioxidative activity, an anticancer activity or an antimicrobial activity, and a water-soluble fullerene.

### Background Art

C60, C70 fullerenes and fullerene with a higher carbon spherical shell structure or tubular structure including chemically modified compounds or complexes thereof, clathrates and the like have been drawing attention as a compound having a bioactivity such as an antioxidative activity, an anticancer activity or an antimicrobial activity.

However, when such a fullerene is actually going to be used, it is very difficult to allow its excellent property such as the above-mentioned bioactivity to be expressed and to make such a property practically available by improving its stability. In addition, it is difficult to solubilize a fullerene in water and more specifically, it is difficult to form an aqueous solution thereof.

In such a circumstance, the inventors of this application have already proposed a novel antioxidant or composition for external use containing a fullerene, a cosmetic material containing the same (e.g., Patent Document 1), and specifically proposed a water-soluble fullerene and an aqueous solution of a fullerene. In particular, from the viewpoint of both the manifestation and stability of the bioactivity, we have proposed a complex of a fullerene or a fullerene mixture with polyvinylpyrrolidone (PVP) as a compound that draws attention.

Patent Document 1: Application number JP-2004-19081

### Disclosure of the invention

Although a PVP/fullerene complex that has been drawing attention as described above, further improvement in its production is also demanded.

The reason is that according to the conventional methods, PVP (polyvinylpyrrolidone) hardly dissolves in an aromatic hydrocarbon such as toluene, xylene, benzene or chlorobenzene, which is known as a solvent for a fullerene or a fullerene mixture, whereby a mixed solution has been prepared by using a halogenated aliphatic hydrocarbon solvent (a solvent containing a -CHX₂ group) such as chloroform, dichloromethane or 1,2- dichloroethane, therefore there is a concern about a problem of the residue of such a halogenated aliphatic hydrocarbon solvent or its environmental load. Accordingly, realization of a method that can produce a PVP/fullerene complex excellent in manifestation of its properties and stability and also excellent in safety and an aqueous solution thereof without using a halogenated aliphatic hydrocarbon solvent has been demanded.

Therefore, in view of the background as described above, an object of the invention of this application is to provide a novel method of producing a PVP/fullerene complex and an aqueous solution thereof under a condition free from a halogen.

In order to achieve the above-mentioned object, the invention of this application provides firstly a method of producing an aqueous solution of a PVP/fullerene complex characterized by mixing an aromatic hydrocarbon solution of a fullerene or a fullerene mixture with an ethanol solution of polyvinylpyrrolidone (PVP), adding water to the mixed solution, removing the solvent, and then obtaining an aqueous solution of a complex of the fullerene or the fullerene mixture with polyvinylpyrrolidone (PVP).

It provides secondly a method of producing an aqueous solution of a PVP/fullerene complex according to the above-mentioned method characterized in that the weight average molecular weight of polyvinylpyrrolidone (PVP) ranges from 6,000 to 1,500,000, thirdly a method of producing an aqueous solution of a PVP/fullerene complex characterized in that the weight ratio (PVP/E) of polyvinylpyrrolidone (PVP) to ethanol (E) in the ethanol solution of polyvinylpyrrolidone (PVP) ranges from 0.01 to 0.5, fourthly a method of producing a PVP/fullerene complex characterized in that the weight ratio (F·A/PVP·E) of the aromatic hydrocarbon solvent solution of a fullerene or a fullerene mixture (F·A) to the ethanol solution of polyvinylpyrrolidone (PVP·E) at the time of mixing ranges from 1.0 to 3.0, and fifthly a method of producing an aqueous solution of a PVP/fullerene complex characterized in that the concentration of a PVP/fullerene complex ranges from 5 to 20% by weight.

It provides sixthly a method of producing a PVP/fullerene complex characterized by evaporating and removing water from an aqueous solution of a PVP/fullerene complex produced by a method according to any one of the invention.

And, it provides seventhly a method of producing an aqueous solution of a PVP/fullerene complex characterized by mixing a PVP/fullerene complex produced by the method according to the above-mentioned sixth invention with water.

### Brief Description of the Drawing

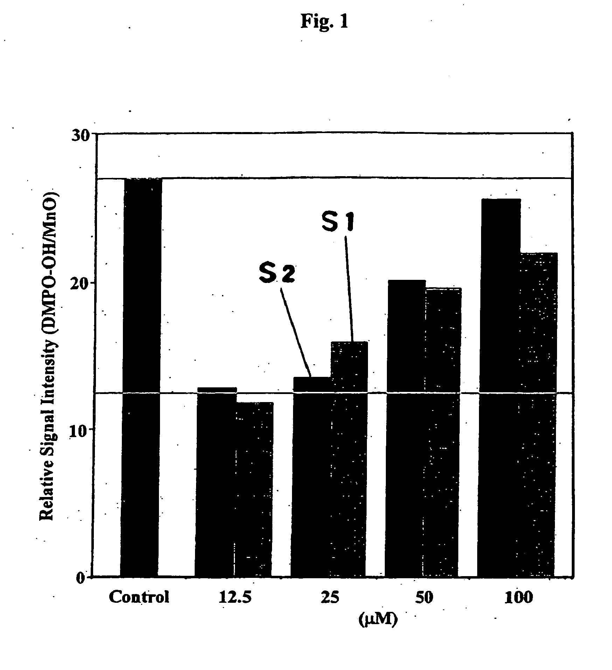

Fig. 1 is a graph showing a free radical scavenging effect measured by ESR (spin-trapping method).

### Best Mode for Carrying Out the Invention

The invention of this application has characteristics as described above, and, its embodiments will be described below.

In the first invention of this application, provided is a method of producing an aqueous solution of a PVP/fullerene complex. In this method, first an aromatic hydrocarbon solvent solution of a fullerene or a fullerene mixture is prepared. Here, the fullerene or the fullerene mixture as a raw material includes C60 or C70 fullerene which has been known so far, a fullerene with a higher carbon spherical shell structure, a variety of fullerenes with a tubular structure such as a carbon nanotube and a fullerene tube or a mixture of two types or more of them. These fullerenes may be a fullerene having a hydroxyl group such as a hydroxylated fullerene, an ether thereof, an acylated fullerene, a metal salt thereof, or a fullerene modified with any of a variety of substituents such as an amino group, a hydrocarbon group or a substituted hydrocarbon group as long as it does not impair the object or effect of the method of the invention of this application. The aromatic hydrocarbon as the solvent for such a fullerene or a fullerene mixture may be any type, however, toluene, xylene, benzene, chlorobenzene, and a mixture of two or more types of them are exemplified as a preferred solvent. Among these, toluene is preferred as the solvent.

In the production of the PVP/fullerene complex and the aqueous solution thereof of the invention of this application, consideration is given that the ratio of the fullerene or the fullerene mixture (F) to the aromatic hydrocarbon solvent (A) is generally set within the range from 0.0005 to 0.0032 (max; maximum solubility) in terms of the weight ratio (F/A). More preferably, the range is from 0.00075 to 0.00095. When the fullerene or the fullerene mixture is excessively contained so that the ratio exceeds 0.0032, it is difficult to form a uniform solution and it becomes difficult to form a uniform solution by mixing it with a PVP ethanol solution. On the contrary, when the ratio is too small, namely less than 0.0005, it is not economical.

The aromatic hydrocarbon solvent solution of a fullerene or a fullerene mixture is mixed with an ethanol solution of polyvinylpyrrolidone (PVP) in the invention of this application. This PVP hardly dissolves in an aromatic hydrocarbon solvent such as toluene, xylene, benzene or chlorobenzene. Therefore, it has been dissolved in a halogenated aliphatic hydrocarbon solvent such as chloroform, dichloromethane or 1,2- dichloroethane so far, however, in the invention of this application, a halogenated aliphatic hydrocarbon solvent (a solvent containing a -CHX₂ group) such as chloroform, dichloromethane or 1,2-dichloroethane is not used at all. Instead, ethanol that is excellent in stability as a pharmaceutically acceptable solvent is used.

PVP (polyvinylpyrrolidone) is a compound represented by the following formula: 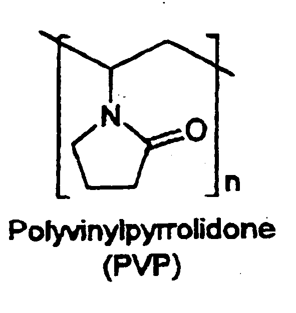 and in the invention of this application, those having a weight average molecular weight (MW) of about 3,00 to 3,000,000, further those having a weight average molecular weight (MW) of about 6,000 to 1,500,000 are preferred. PVP may be either synthesized or commercially obtained. As the ratio of such PVP to ethanol (E), the range from 0.01 to 0.5 in terms of the weight ratio (PVP/E) is generally considered. More preferably, the range is from 0.125 to 0.25. When the weight ratio (PVP/E) is less than 0.01, it is necessary to maintain extremely high stirring efficiency during the reaction; therefore it is not preferred. On the contrary, when it exceeds 0.5, the reaction efficiency is significantly decreased due to the high viscosity of the solution; therefore, it is not preferred. In order to obtain a uniform solution by mixing it with the aromatic hydrocarbon solvent solution of a fullerene or a fullerene mixture, it is preferred to hold the water content in the solvent ethanol (E) to about 0.5% by weight or less.

As for the mixing of the aromatic hydrocarbon solution of a fullerene or a fullerene mixture (F·A) with the PVP ethanol solution (PVP·E), consideration is given that the ratio is set within the range from 1.0 to 3.0 in terms of the weight ratio (F·A/PVP·E). More preferably, the range is preferablly from 1.5 to 2.0. When the weight ratio is less than 1.0, the reaction efficiency is decreased; therefore, it is not preferred. On the contrary, when it exceeds 3.0, the removal of the solvent is inefficient; therefore it is not preferred.

It is preferred that the mixing of both solutions is generally carried out while stirring the solutions at a temperature ranging from 15°C to 25°C. The stirring for this operation is carried out, for example, under the condition that the rotation speed of a blade propeller is about 400 rpm for about 12 to 15 hours.

By mixing both solutions as described above, a uniform mixed solution is formed.

In the invention of this application, by subsequently performing the steps of adding water to the mixed solution and removing the solvent, an aqueous solution of a PVP/fullerene complex is formed; however, various methods and procedures may be adopted for these steps.

For example, a procedure in which the mixed solution is dried by evaporation, a suspension is prepared by adding water thereto, then evaporation is performed and a solvent such as toluene is removed by azeotropic distillation to dryness, and water is added again to the dried substance to form an aqueous solution is carried out. Alternatively, a procedure in which water is added to the above-mentioned mixed solution, evaporation is performed followed by azeotropic distillation, and then water is added thereto again may be carried out.

In any procedure, consideration is given so that the amount of added water is adjusted such that the concentration of the PVP/fullerene complex in the final aqueous solution of the PVP/fullerene complex falls in the range from 5 to 20% by weight. More preferred concentration of the PVP/fullerene complex in the aqueous solution ranges from 6.5 to 14.5% by weight.

When the concentration of the PVP/fullerene complex in the aqueous solution is less than 5% by weight, the evaporation to dryness is inefficient; therefore it is not preferred. On the contrary, when it exceeds 20% by weight, it is necessary to increase the solubility by heating at the time of filtration because of the high viscosity of the aqueous solution; therefore it is not preferred.

Of course, the concentration within the range may be adjusted by the final addition of water according to the application of the aqueous solution.

In addition, it is preferred that the temperature of the water to be added is set within the range from about 20°C to 35°C.

The aqueous solution of a PVP/fullerene complex of the invention of this application produced by for example the above-mentioned method can be formed into a solid PVP/fullerene complex by evaporation to dryness. Further, it is dissolved in water to form an aqueous solution, which can be used in various applications.

Accordingly, hereinafter, more detail description will be made by showing Example. Of course, the invention is not limited to the following Example.

### Example

<A> A solution of toluene (20 ml) containing 16.0 mg of MIX (mixture) fullerene (average molecular weight of 744: powder) containing C60 fullerene and C70 fullerene and a solution of ethanol (10 ml) containing 2.0 g of PVP having a weight average molecular weight of 60,000 were prepared respectively, and both solutions were mixed at room temperature and subjected to stirring for 12 hours. In this way, a uniform mixed solution was obtained.
   Then, this uniform mixed solution was dried by evaporation and added with 30 ml of water to form a suspension.
   This suspension was treated by evaporation, and toluene was removed by azeotropic distillation to dryness. Then, 30 ml of water was added thereto, and sonication was carried out for 15 minutes. In this way, an aqueous solution of a PVP/fullerene complex was obtained. The solution was filtered (0.80 µm filter), whereby a final aqueous solution of a PVP/fullerene complex was obtained.
   The obtained aqueous solution has a high transparency, and a small amount of black precipitate which is usually generated when a solution obtained by using chloroform as a solvent is left all day and night at room temperature was not observed at all.
   In addition, the above-mentioned aqueous solution was dried by evaporation, ground, and dried under vacuum (12 hours), whereby a PVP/fullerene complex powder was obtained.
<B> As for the obtained aqueous solution of a PVP/fullerene complex (S1), its free radical scavenging effect was measured by ESR (spin-trapping method). The comparison of the solution S1 with an aqueous solution (S2) prepared by using a chloroform solvent is shown in Fig. 1. It was confirmed that as shown in Fig. 1, a free radical scavenging effect equal to or more than that of S2 is obtained.

ESR analysis (spin-trapping method) using a spin-trapping agent is a very useful method for detecting free radicals in which highly reactive and unstable free radicals are reacted with a spin-trapping agent and the ESR spectrum of the generated stable spin adduct is measured at room temperature.

In this method, as for the hydroxyl radical generated by the reaction of hydrogen peroxide with ferrous sulfate (Fenton reaction), 5,5-dimethyl-1-pyrroline-N-oxide (DMPO) is used as the spin-trapping agent, the signal of DMPO-OH generated by the reaction of DMPO and the hydroxyl radical is measured (Control).

The vertical axis of Fig. 1 indicates the relative value of DMPO-OH to the internal standard (MnO), and the lateral axis indicates the concentration of the given PVP/fullerene complex. Control is the maximum value (26.5) of DMPO-OH generated and the lower limit (12.5) is the value in the case where hydroxyl radical (free radical) is completely trapped (scavenged) by dimethylsulfoxide (DMSO). It was confirmed that as shown in Fig. 1, a scavenging effect on hydroxyl radical (free radical) can be equally obtained in any concentration of the aqueous solution of a PVP/fullerene complex (S1) and the aqueous solution prepared by using a chloroform solvent (S2). In particular, a significant free radical scavenging effect was observed at a concentration ranging from 12.5 to 25 µM.

### Industrial Applicability

According to the invention of this application as described above, without using a halogenated aliphatic hydrocarbon solvent for which there is a concern about an effect of the residue on the human body or an increase in the environmental load of its disposal, a PVP/fullerene complex and an aqueous solution thereof can be produced by using ethanol as a pharmaceutically acceptable solvent under a condition free from a halogen. The produced PVP/fullerene complex is excellent in stability and safety as well as manifestation of its properties such as a bioactivity.

## Claims

1. A method of producing an aqueous solution of a PVP/fullerene complex **characterized by** mixing an aromatic hydrocarbon solution of a fullerene or a fullerene mixture with an ethanol solution of polyvinylpyrrolidone (PVP), adding water to the mixed solution; removing the solvent, and then obtaining an aqueous solution of a complex of the fullerene or the fullerene mixture with polyvinylpyrrolidone (PVP).

2. The method of producing an aqueous solution of a PVP/fullerene complex according to Claim 1, **characterized in that** the weight average molecular weight of polyvinylpyrrolidone (PVP) ranges from 6,000 to 1,500,000.

3. The method of producing an aqueous solution of a PVP/fullerene complex according to Claim 1 or 2, **characterized in that** the weight ratio (PVP/E) of polyvinylpyrrolidone (PVP) to ethanol (E) in the ethanol solution of polyvinylpyrrolidone (PVP) ranges from 0.01 to 0.5.

4. The method of producing an aqueous solution of a PVP/fullerene complex according to any one of Claims 1 to 3, **characterized in that** the weight ratio (F·A/PVP·E) of the aromatic hydrocarbon solvent solution of a fullerene or a fullerene mixture (F·A) to the ethanol solution of polyvinylpyrrolidone (PVP·E) at the time of mixing ranges from 1.0 to 3.0.

5. The method of producing an aqueous solution of a PVP/fullerene complex according to any one of Claims 1 to 4, **characterized in that** the concentration of the PVP/fullerene complex ranges from 5 to 20% by weight.

6. A method of producing a PVP/fullerene complex **characterized by** evaporating and removing water from an aqueous solution of a PVP/fullerene complex produced by the method according to any one of Claims 1 to 5.

7. A method of producing an aqueous solution of a PVP/fullerene complex **characterized by** mixing a PVP/fullerene complex produced by the method according to Claim 6 with water.

## Patentansprüche

1. Verfahren zur Herstellung einer wässrigen Lösung eines PVP/Fulleren-Komplexes, **dadurch gekennzeichnet, dass** man eine aromatische Kohlenwasserstofflösung eines Fullerens oder eines Fulleren-Gemisches mit einer Ethanollösung von Polyvinylpyrrolidon (PVP) mischt, die gemischte Lösung mit Wasser versetzt; das Lösungsmittel entfernt und eine wässrige Lösung eines Komplexes des Fullerens oder des Fullerengemisches mit Polyvinylpyrrolidon (PVP) erhält.

2. Verfahren zur Herstellung einer wässrigen Lösung eines PVP/Fulleren-Komplexes nach Anspruch 1, **dadurch gekennzeichnet, dass** das gewichtsmittlere Molekulargewicht des Polyvinylpyrrolidons (PVP) 6.000 bis 1.500.000 beträgt.

3. Verfahren zur Herstellung einer wässrigen Lösung eines PVP/Fulleren-Komplexes nach Anspruch 1 oder 2, **dadurch gekennzeichnet, dass** das Gewichtsverhältnis (PVP/E) des Polyvinylpyrrolidons (PVP) zum Ethanol (E) in der Ethanollösung des Polyvinylpyrrolidons (PVP) 0,01 bis 0,5 beträgt.

4. Verfahren zur Herstellung einer wässrigen Lösung eines PVP/Fulleren-Komplexes nach einem der Ansprüche 1 bis 3, **dadurch gekennzeichnet, dass** das Gewichtsverhältnis (F·A/PVP·E) der aromatischen Kohlenwasserstofflösung eines Fullerens oder eines Fulleren-Gemisches (F·A) zur Ethanollösung des Polyvinylpyrrolidons (PVP·E) zum Zeitpunkt des Mischens 1,0 bis 3,0 beträgt.

5. Verfahren zur Herstellung einer wässrigen Lösung eines PVP/Fulleren-Komplexes nach einem der Ansprüche 1 bis 4, **dadurch gekennzeichnet, dass** die Konzentration des PVP/Fulleren-Komplexes 5 bis 20 Gew.-% beträgt.

6. Verfahren zur Herstellung eines PVP/Fulleren-Komplexes, **dadurch gekennzeichnet, dass** man Wasser aus einer wässrigen Lösung eines PVP/Fulleren-Komplexes, hergestellt nach einem Verfahren nach einem der Ansprüche 1 bis 5, abdampft und entfernt.

7. Verfahren zur Herstellung einer wässerigen Lösung eines PVP/Fulleren-Komplexes, **dadurch gekennzeichnet, dass** man einen nach dem Verfahren nach Anspruch 6 hergestellten PVP/Fulleren-Komplex mit Wasser mischt.

## Revendications

1. Procédé de production d'une solution aqueuse contenant un complexe de PVP/fullerène **caractérisé par** le mélange d'une solution contenant un fullerène ou un mélange de fullerènes dans un hydrocarbure aromatique avec une solution de polyvinylpyrrolidone (PVP) dans l'éthanol, l'ajout d'eau à la solution mélangée ; l'élimination du solvant, puis l'obtention d'une solution aqueuse contenant un complexe du fullerène ou du mélange de fullerène avec la polyvinylpyrrolidone (PVP).

2. Procédé de production d'une solution aqueuse contenant un complexe de PVP/fullerène selon la revendication 1, **caractérisé en ce que** le poids moléculaire moyen en poids de la polyvinylpyrrolidone (PVP) est situé dans la plage allant de 6 000 à 1 500 000.

3. Procédé de production d'une solution aqueuse contenant un complexe de PVP/fullerène selon la revendication 1 ou 2, **caractérisé en ce que** le rapport en poids (PVP/E) entre la polyvinylpyrrolidone (PVP) et l'éthanol (E) dans la solution de polyvinylpyrrolidone (PVP) dans l'éthanol se situe dans la plage allant de 0,01 à 0,5.

4. Procédé de production d'une solution aqueuse contenant un complexe de PVP/fullerène selon l'une quelconque des revendications 1 à 3, **caractérisé en ce que** le rapport en poids (F·A/PVP·E) entre la solution contenant un fullerène ou un mélange de fullerènes dans un solvant de type hydrocarbure aromatique (F·A) et la solution contenant la polyvinylpyrrolidone dans l'éthanol (PVP·E) au moment du mélange se situe dans la plage allant de 1,0 à 3,0.

5. Procédé de production d'une solution aqueuse contenant un complexe de PVP/fullerène selon l'une quelconque des revendications 1 à 4, **caractérisé en ce que** la concentration du complexe de PVP/fullerène se situe dans la plage allant de 5 % en poids à 20 % en poids.

6. Procédé de production d'un complexe de PVP/fullerène **caractérisé par** l'évaporation et l'élimination de l'eau d'une solution aqueuse contenant un complexe de PVP/fullerène produite par le procédé selon l'une quelconque des revendications 1 à 5.

7. Procédé de production d'une solution aqueuse contenant un complexe de PVP/fullerène, **caractérisé par** le mélange d'un complexe de PVP/fullerène produit par le procédé selon la revendication 6 avec de l'eau.
